# EUROPÄISCHE PATENTSCHRIFT

(11) **EP 3 903 689 B1**
(45) Veröffentlichungstag und Bekanntmachung des Hinweises auf die Patenterteilung: **22.10.2025**
(21) Anmeldenummer: 21181118.7
(22) Anmeldetag: 28.03.2017
(51) Int. Cl.: A61B 17/00, G01D 5/14

(54) **SENSORISCHES GEBERSYSTEM**
SENSOR-BASED ENCODER SYSTEM
SYSTÈME DE CAPTEUR SENSORIEL

(30) Priorität: 30.03.2016 DE 102016105789
(43) Veröffentlichungstag der Anmeldung: 03.11.2021
(62) Teilanmeldung aus: 17714195.9
(73) Patentinhaber: Aesculap AG, 78532 Tuttlingen (DE)
(72) Erfinder: KAHLER, Thomas, 78606 Seitingen-Oberflacht (DE); HÖGERLE, Roland-Alois, 78532 Tuttlingen (DE); MACHILL, Martin, 78604 Rietheim-Weilheim (DE)
(74) Vertreter: Winter, Brandl - Partnerschaft mbB

(56) Entgegenhaltungen:
- EP-A1- 2 923 651
- EP-A1- 2 992 842
- WO-A2-2007/002180
- US-A1- 2012 116 391
- US-A1- 2014 200 594

## Beschreibung

Die Erfindung betrifft ein sensorisches Gebersystem und bezieht sich insbesondere auf ein sensorisches Gebersystem für ein medizinisches, insbesondere chirurgisches, Motorensystem oder einen medizinischen, insbesondere chirurgischen, elektrischen Antrieb.

Ein bekanntes sensorisches Gebersystem für ein chirurgisches Motorensystem ist ein einkanaliges, nicht redundantes Gebersystem unter Verwendung beispielsweise eines Hallsensors für elektrische Systeme, das unter anderem in einem ebenfalls an sich bekannten Motorkabel mit Handsteuerung Verwendung findet. Bei solchen elektrischen Systemen werden bislang mechanische Rückholfedern eingesetzt, die eine Betätigungseinrichtung wie beispielsweise einen Hebel oder einen Schieber nach erfolgter Betätigung durch einen Anwender in die Ausgangstellung zurückführen. Wird ungewollt ein falsches Signal von einem Sensor des Gebersystems ausgegeben, kann es zu einer sicherheitsrelevanten Funktionsstörung kommen. Falls beispielsweise der Sensor plötzlich ausfallen sollte, etwa aufgrund eines Stuck-at-Fault-Fehlers bzw. Haftfehlers des Sensors (ein Fehler, bei dem etwa ein Sensorgatter an einem Eingang oder Ausgang auf einem bestimmten Wert "feststeckt"), kann ein Anwendungsteil oder Werkzeug in Gang geraten bzw. starten, ohne dass ein Anwender die Betätigungseinrichtung betätigt. Dadurch kann der Anwender, der mit dem Anwendungsteil und/oder Werkzeug beispielsweise in bzw. an einem Situs ist, überrascht werden. Nachteilig ist somit, dass bei dem nicht redundanten Gebersystem ein Elektronikausfall nicht abgesichert ist.

Die EP 2 923 651 A1 offenbart ein chirurgisches Instrument, das vorgesehen und ausgebildet ist, Gewebe zu verbinden und zu schneiden und weist zwei Hallsensoren auf, welche eine Position eines Hebels, welcher an einen Sender gekoppelt ist, detektierten können.

Die US 2014 / 020 0594 A1 offenbart ein chirurgisches Instrument mit einem Schalter, welcher einen in seiner Position durch einen Benutzer manipulierbaren Magnet und eine Vielzahl an Hallsensoren aufweist.

Der Erfindung liegt daher als eine Aufgabe zugrunde, ein sensorisches Gebersystem für ein medizinisches oder chirurgisches Motorensystem oder einen medizinischen oder chirurgischen elektrischen Antrieb dahingehend zu verbessern, dass eine Abfrage auf Plausibilität der Signale ermöglicht und Sicherheit des Systems erhöht wird.

Diese Aufgabe wird erfindungsgemäß durch eine Handsteuereinrichtung mit den Merkmalen des Anspruchs 1 gelöst. Vorteilhafte Weiterbildungen der Erfindung sind Gegenstand der beigefügten Unteransprüche.

Der Erfindung liegt die allgemeine Idee zugrunde, in Handsteuerungen mehrkanalige und damit redundante Signale und/oder Gebersysteme vorzusehen. Durch Verwendung von zumindest zwei Sensoren, die unabhängig voneinander, jedoch parallel (zeitgleich) angesteuert werden, wird sichergestellt, dass bei Ausfall zumindest eines Sensors auf einen Fehlerfall oder Defekt erkannt und die Handsteuerung durch eine externe Steuereinheit, mit der die Handsteuerung in der Regel verbunden ist, deaktiviert werden kann. Gemäß der zugrundeliegenden allgemeinen Idee können dazu die Ausgangssignale oder Ausgangssignalwerte der zumindest zwei Sensoren gleich oder unterschiedlich programmiert werden, kann die Ansteuerung der Sensoren vorzugsweise mittels eines Magnetfelds bzw. magnetisch und in diesem Fall mittels eines Magnets für alle Sensoren, mittels mehreren Magneten für jeweilige Sensorgruppen oder mittels einzelnen Magneten für jeden jeweiligen Sensor erfolgen. Außerdem können der Magnet bzw. die Magnete und/oder der Sensor bzw. die Sensoren zugunsten von Wasser- und/oder Dampfdichtheit gekapselt, beispielsweise vergossen, sein. Ferner können der Sensor bzw. die Sensoren in einer Vorrichtung feststehend angeordnet sein und sich der Magnet bzw. die Magnete auf den Sensor bzw. die Sensoren zu bewegen, beispielsweise kreisbogenförmig bei etwa Anordnung einer Scharniervorrichtung oder linear. Eine Beschränkung auf eine Bewegungsbahn und/oder Bewegungsrichtung besteht jedoch nicht, grundsätzlich sind Bewegungen in beliebige Richtungen und/oder auf beliebigen Bewegungsbahnen darstellbar.

Schließlich können mehrere und zumindest zwei unabhängige Sensoren in einem Baustein mit mehreren bzw. zumindest zwei unabhängigen, integrierten Sensorbereichen integriert sein.

Die Verwendung eines redundanten sensorischen Gebersystems bei Hand- und/oder Fußsteuerungen bietet eine erhöhte Sicherheit gegen ein anforderungsloses und ungewolltes Starten eines Anwendungsteils oder Werkzeugs und verhindert dadurch ein Anlaufen entsprechender Einrichtungen und somit Verletzungen von empfindlichem Gewebe, wenn sich das Werkzeug im Situs befindet.

Im Einzelnen beinhaltet ein sensorisches Gebersystem für ein medizinisches Motorensystem eine mehrkanalig redundant ausgelegte Erfassungseinrichtung, die in einer zur Steuerung des medizinischen Motorensystems angepassten Handsteuereinrichtung aufgenommen und dazu angeordnet ist, die Betätigung einer Betätigungseinrichtung der Handsteuereinrichtung zu erfassen, wobei die mehrkanalig redundante Erfassungseinrichtung auf Plausibilität von Ausgangssignalen derselben abfragbar ausgelegt ist.

Die mehrkanalig redundante Erfassungseinrichtung beinhaltet zumindest zwei Erfassungsempfangseinrichtungen und eine Erfassungssendeeinrichtung.

Bevorzugt sind die zumindest zwei Erfassungsempfangseinrichtungen gleichartige Erfassungsempfangseinrichtungen.

Die zumindest eine Erfassungssendeeinrichtung beinhaltet genau eine Erfassungssendeeinrichtung, die allen der zumindest zwei Erfassungsempfangseinrichtungen zugeordnet ist und dazu angeordnet ist, diese mit einer dem Grunde nach gleichen Erfassungssendegröße zu beaufschlagen.

Alternativ ist für jede der zumindest zwei Erfassungsempfangseinrichtungen eine zugeordnete Erfassungssendeeinrichtung vorgesehen, die dazu angeordnet ist, eine entsprechende der zumindest zwei Erfassungsempfangseinrichtungen mit einer jeweiligen Erfassungssendegröße zu beaufschlagen.

Bevorzugt sind als die zumindest zwei Erfassungsempfangseinrichtungen zumindest zwei Hall-Sensoren angeordnet und ist als die zumindest eine Erfassungssendeeinrichtung zumindest ein Magnet angeordnet.

Alternativ bevorzugt sind als die zumindest zwei Erfassungsempfangseinrichtungen zumindest zwei Lichtsensoren angeordnet und ist als die zumindest eine Erfassungssendeeinrichtung zumindest eine Lichtquelle angeordnet.

Alternativ bevorzugt sind die zumindest zwei Erfassungsempfangseinrichtungen unterschiedliche Erfassungsempfangseinrichtungen.

Bevorzugt sind im letztgenannten Fall zwei Erfassungsempfangseinrichtungen und zwei Erfassungssendeeinrichtungen angeordnet, wobei eine der zwei Erfassungsempfangseinrichtungen ein Hall-Sensor ist und die andere der zwei Erfassungsempfangseinrichtungen ein Lichtsensor ist, und eine der zwei Erfassungssendeeinrichtungen ein Magnet ist, der dem Hall-Sensor zugeordnet ist, und die andere der zwei Erfassungssendeeinrichtungen eine Lichtquelle ist, die dem Lichtsensor zugeordnet ist.

Bevorzugt sind die zumindest zwei Erfassungsempfangseinrichtungen mit jeweils voneinander unabhängigen Erfassungsbereichen in einer gemeinsamen Gehäuseanordnung aufgenommen.

Die zumindest zwei Erfassungsempfangseinrichtungen und die eine oder die jeweils eine zugeordnete Erfassungssendeeinrichtung sind dampf- und/oder wasserdicht gekapselt.

Bevorzugt sind die zumindest zwei Erfassungsempfangseinrichtungen ortsfest angeordnet, und ist die zumindest eine Erfassungssendeeinrichtung beweglich angeordnet und durch Betätigung der Betätigungseinrichtung auf einer vorbestimmten Bahnkurve auf die zumindest zwei Erfassungsempfangseinrichtungen zu und von dieser weg bewegbar.

Bevorzugt sind die zumindest zwei Erfassungsempfangseinrichtungen dazu programmiert, dasselbe Ausgangssignal auszugeben, oder sind alternativ dazu programmiert, unterschiedliche Ausgangssignale auszugeben.

Vorteilhafterweise beinhaltet eine Handsteuereinrichtung für ein medizinisches Motorensystem ein wie vorstehend beschrieben konfiguriertes Gebersystem.

In anderen Worten ausgedrückt wird die Aufgabe gelöst durch ein Gebersystem für ein medizinisches Motorensystem, mit einer mehrkanalig redundant ausgelegten Erfassungseinrichtung, die in einer zur Steuerung des medizinischen Motorensystems angepassten Handsteuereinrichtung aufgenommen und dazu angeordnet ist, die Betätigung einer Betätigungseinrichtung der Handsteuereinrichtung zu erfassen, wobei die Betätigungseinrichtung als Hebel oder Schieber konfiguriert ist;
die mehrkanalig redundante Erfassungseinrichtung zumindest zwei Erfassungsempfangseinrichtungen und genau eine Erfassungssendeeinrichtung beinhaltet; die genau eine Erfassungssendeeinrichtung allen der zumindest zwei Erfassungsempfangseinrichtungen zugeordnet ist und dazu angeordnet ist, diese mit einer dem Grunde nach gleichen Erfassungssendegröße zu beaufschlagen; und die mehrkanalig redundante Erfassungseinrichtung auf Plausibilität von Ausgangssignalen derselben abfragbar ausgelegt ist.

Das Gebersystem ist so weitergebildet, dass die zumindest zwei Erfassungsempfangseinrichtungen und die genau eine oder die jeweils eine Erfassungssendeeinrichtung dampf- und/oder wasserdicht gekapselt sind.

Vorzugsweise ist das Gebersystem so weitergebildet, dass die zumindest zwei Erfassungsempfangseinrichtungen in Richtung eines Hebelwegs oder Schieberwegs der genau einen Erfassungssendeeinrichtung versetzt angeordnet sind.

Vorzugsweise ist das Gebersystem so weitergebildet, dass die zumindest zwei Erfassungsempfangseinrichtungen Hall-Sensoren oder Lichtsensoren und die genau eine Erfassungssendeeinrichtung einen Magneten oder eine Lichtquelle beinhalten.

Vorzugsweise ist das Gebersystem so weitergebildet, dass die zumindest zwei Erfassungsempfangseinrichtungen gleichartige Erfassungsempfangseinrichtungen sind.

Vorzugsweise ist das Gebersystem so weitergebildet, dass für jede der zumindest zwei Erfassungsempfangseinrichtungen eine zugeordnete Erfassungssendeeinrichtung vorgesehen und dazu angeordnet ist, eine entsprechende der zumindest zwei Erfassungsempfangseinrichtungen mit einer jeweiligen Erfassungssendegröße zu beaufschlagen.

Vorzugsweise ist das Gebersystem so weitergebildet, dass die zumindest zwei Erfassungsempfangseinrichtungen unterschiedliche Erfassungsempfangseinrichtungen sind.

Vorzugsweise ist das Gebersystem so weitergebildet, dass die zumindest zwei Erfassungsempfangseinrichtungen mit jeweils voneinander unabhängigen Erfassungsbereichen in einer gemeinsamen Gehäuseanordnung aufgenommen sind.

Vorzugsweise ist das Gebersystem so weitergebildet, dass die zumindest zwei Erfassungsempfangseinrichtungen ortsfest angeordnet sind, und die genau eine Erfassungssendeeinrichtung beweglich angeordnet und durch Betätigung der Betätigungseinrichtung auf einer vorbestimmten Bahnkurve auf die zumindest zwei Erfassungsempfangseinrichtungen zu bewegbar ist.

Vorzugsweise ist das Gebersystem so weitergebildet, dass die zumindest zwei Erfassungsempfangseinrichtungen dazu programmiert sind, dasselbe Ausgangssignal auszugeben, oder dazu programmiert sind, unterschiedliche Ausgangssignale auszugeben.

Des Weiteren wird die Aufgabe gelöst durch eine Handsteuereinrichtung für ein medizinisches Motorensystem, mit einem Gebersystem gemäß vorstehender Definition.

Die Erfindung wird nachstehend mit weiteren Vorteilen und Wirkungen anhand bevorzugter Ausführungsbeispiele unter Bezugnahme auf die Zeichnung näher beschrieben. Es zeigen:
Fig. 1 eine schematische Darstellung einer Handsteuereinrichtung eines medizinischen oder chirurgischen Motorensystems mit Hebelbetätigung, die ein sensorisches Gebersystem gemäß einem von hierin beschriebenen Ausführungsbeispielen beinhaltet;
Fig. 2 eine schematische Darstellung einer Handsteuereinrichtung eines medizinischen oder chirurgischen Motorensystems mit Schieberbetätigung, die ein sensorisches Gebersystem gemäß einem von hierin beschriebenen Ausführungsbeispielen beinhaltet;
Fig. 3 eine Prinzipdarstellung von Anordnungen am Beispiel eines auf Magneten und magnetisch beeinflussbaren Sensoren basierenden sensorischen Gebersystems; und
Fig. 4 eine Prinzipdarstellung von Konzepten für Handsteuereinrichtungen unter Verwendung der Anordnungen nach Fig. 3.

Gleiche oder funktional äquivalente Merkmale sind in den einzelnen Figuren mit denselben Bezugszeichen versehen und werden zweckmäßig nicht mehrfach beschrieben.

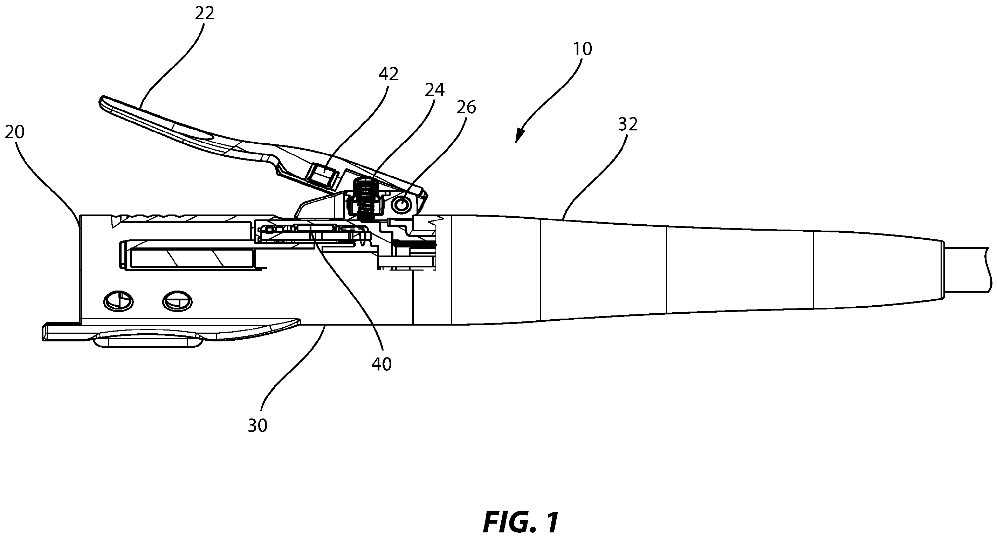

Fig. 1 zeigt eine schematische Darstellung einer Handsteuereinrichtung 10 eines medizinischen oder chirurgischen Motorensystems mit Hebelbetätigung, die ein sensorisches Gebersystem gemäß einem von hierin beschriebenen Ausführungsbeispielen beinhaltet.

Eine solche Handsteuereinrichtung 10 ist mit Ausnahme der Unterschiede gemäß einem der nachfolgenden Ausführungsbeispiele dem Grunde nach bekannt und umfasst im Wesentlichen einen anwendungsseitigen Motorkabelstecker 20 zur Versorgung und Ansteuerung des beispielsweise in das Handstück integrierten elektronischen Motorensystems über etwa eine (nicht dargestellte) externe Steuereinheit, einen handbetätigbaren Hebel 22, der mittels einer mechanischen Rückholfeder 24, die dazu dient, den unbeaufschlagten Hebel 22 in die Ausgangstellung zurückzuführen, federbelastet und an einer Hebelachse 26 an einem Gehäusekörper 30 der Handsteuereinrichtung 10 verschwenkbar angelenkt ist. Abtriebsseitig befindet sich eine Kabelanordnung mit Knickschutzhülle 32, an welchem ein von dem Motor des Motorsystems angetriebenes medizinisches Werkzeug wie beispielsweise Fräser zur Behandlung im Situs in Eingriff steht. Einsatzgebiete derartiger Systeme können beispielsweise Knochenchirurgie und Arthroskopie, Neurochirurgie, die Veterinärmedizin und dergleichen sein, ohne dass hierauf eine Beschränkung besteht.

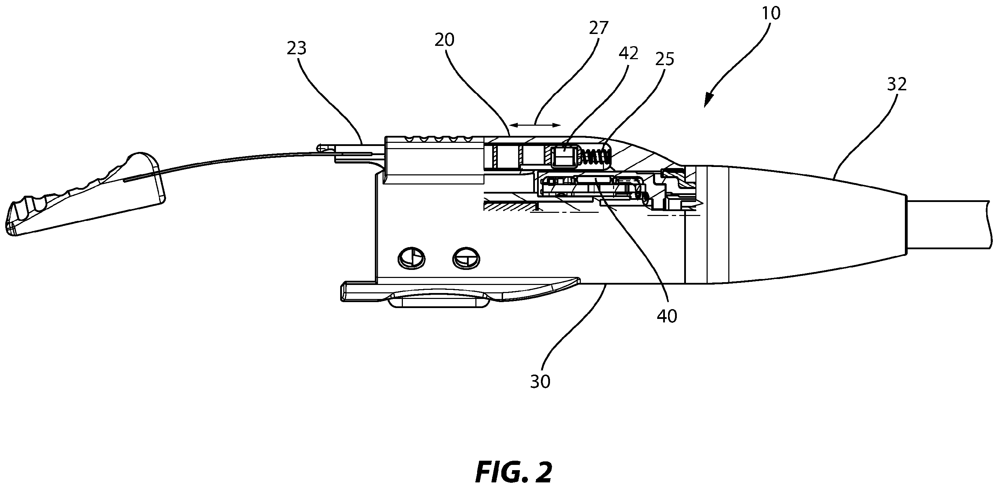

Fig. 2 zeigt eine schematische Darstellung einer Handsteuereinrichtung eines medizinischen oder chirurgischen Motorensystems mit Schieberbetätigung, die ein sensorisches Gebersystem gemäß einem von hierin beschriebenen Ausführungsbeispielen beinhaltet.

Im Unterschied zu der Handsteuereinrichtung nach Fig. 1 ist bei der Ausführungsform nach Fig. 2 anstelle des Hebels 22 ein Schieber 23 zur manuellen Aktivierung des elektronischen Motorensystems vorgesehen, der mittels einer mechanischen Rückholfeder 25, die dazu dient, den unbeaufschlagten Schieber 23 in die Ausgangstellung zurückzuführen, federbelastet und entlang eines Verschiebewegs 27 an einem Gehäusekörper 30 der Handsteuereinrichtung 10 verfahrbar angelenkt ist.

In Übereinstimmung mit den vorliegenden Ausführungsbeispielen weist die Handsteuereinrichtung 10 nach Fig. 1 oder Fig. 2 darüber hinaus ein Gebersystem für das medizinische oder chirurgische Motorensystem auf, das eine mehrkanalig redundant ausgelegte Erfassungseinrichtung beinhaltet, die in der zur Steuerung des medizinischen Motorensystems angepassten Handsteuereinrichtung 10 aufgenommen und dazu angeordnet ist, die Betätigung des Hebels 22 oder des Schiebers 23 der Handsteuereinrichtung 10 zu erfassen, wobei die mehrkanalig redundante Erfassungseinrichtung auf Plausibilität von Ausgangssignalen von in dem Gebersystem angeordneten Sensoren 40 abfragbar ausgelegt ist.

Im Einzelnen beinhaltet die mehrkanalig redundante Erfassungseinrichtung dazu gemäß den vorliegenden Ausführungsbeispielen zumindest zwei Sensoren 40, die jeweils Erfassungsempfangseinrichtungen bilden und als Hall-Sensoren und/oder optische Sensoren in Form von beispielsweise Lichtsensoren ausgebildet sein können, und zumindest einen die Sensoren 40 beaufschlagenden Größengeber oder (Größen)Sender 42, der eine Erfassungssendeeinrichtung bildet und entsprechend als ein Magnet mit jeweils magnetischem Nord- und Südpol bzw. als im sichtbaren Bereich oder nicht sichtbaren Bereich, beispielsweise Infrarotbereich, Licht emittierende Lichtquelle ausgebildet sein kann.

Die zumindest zwei Sensoren 40 und der zumindest eine Sender 42 bilden ein mehrkanaliges (redundantes) (Signal)Gebersystem, das dazu dient, die Handsteuereinrichtung 10 gegen ein Loslaufen des Werkzeugs ohne Anforderung des Anwenders abzusichern. In anderen Worten ist die Gesamtanordnung dazu ausgelegt, mittels des mehrkanaligen redundanten Gebersystems ein Starten oder Loslaufen eines Anwendungsteils oder Werkzeugs zu unterbinden, wenn ein Fehlerfall erfasst wird und/oder ohne dass von einem Anwender der Hebel 22 oder der Schieber 23 betätigt wird. Daher kann auch in einem Fall, in welchem einer der Sensoren 40 unvermittelt defekt wird, eine Zustands- bzw. Signalplausibilitätsabfrage auf der Grundlage der zumindest zwei Ausgangssignale der zumindest zwei Sensoren 40 ein Fehlerzustand erkannt und durch Auslösen einer Abschaltung der Handsteuereinrichtung 10 verhindert werden, dass der Anwender mit einem Anwendungsteil/Werkzeug im Situs von einem plötzlichen Loslaufen des Werkzeugs überrascht wird. Durch Verwendung zumindest zweier Sensoren 40, die unabhängig voneinander, jedoch parallel (zeitgleich) angesteuert werden, wird somit sichergestellt, dass bei Ausfall eines Sensors 40 der Defekt erkannt und die Handsteuereinrichtung 10 durch die Steuereinheit deaktiviert werden kann.

Die zumindest zwei Sensoren 40 können mit entsprechend angepasster Zustands- oder Signalplausibilitätsabfrage zur Ausgabe grundlegend desselben Ausgangssignals eingerichtet sein, beispielsweise wenn die zumindest zwei Sensoren 40 gleichartig oder von gleicher Art bzw. Bauart sind, oder alternativ zur Ausgabe unterschiedlicher Ausgangssignale eingerichtet sein, beispielsweise wenn die zumindest zwei Sensoren 40 ungleichartiger oder ungleicher Art bzw. Bauart sind. Geben beispielsweise die einzelnen Sensoren 40 jeweils Ausgangssignale aus, die in ihrer Kombination von vorbestimmten plausiblen Zuständen oder Plausibilitätsbedindungen abweichen, kann auf das Vorliegen eines Fehlerzustands geschlossen werden.

In einem von Ausführungsbeispielen kann genau ein Sender 42 angeordnet sein, der allen der zumindest zwei Sensoren 40 zugeordnet ist und dazu angeordnet ist, diese mit einer dem Grunde nach gleichen Erfassungssendegröße zu beaufschlagen. In diesem Fall empfangen oder erfassen beide Sensoren 40 das dem Grunde nach gleiche Signal bzw. eine entsprechende Größe von ein und demselben Sender 42, beispielsweise einem Magneten oder einer Lichtquelle.

Alternativ kann für jede der zumindest zwei Sensoren 40 jeweils ein jedem Sensor 40 einzeln zugeordneter Sender 42 vorgesehen sein, der dazu angeordnet ist, den entsprechenden ihm zugeordneten Sensor 42 mit seinem jeweiligen Signal oder seiner jeweiligen Größe zu beaufschlagen.

In einem Ausführungsbeispiel sind als die zumindest zwei Sensoren 40 zumindest zwei Hall-Sensoren angeordnet und ist als der zumindest eine Sender 42 zumindest ein Magnet angeordnet. In einem anderen Ausführungsbeispiel sind als die zumindest zwei Sensoren 40 zumindest zwei Lichtsensoren angeordnet und ist als der zumindest eine Sender 42 zumindest eine Lichtquelle angeordnet.

In dem Fall, in dem die zumindest zwei Sensoren 40 von unterschiedlicher Bauart sind bzw. dazu angeordnet sind, unterschiedliche (physikalische) Größen zu erfassen, können in einem anderen Ausführungsbeispiel zwei Sensoren 40 und zwei Sender 42 angeordnet sein, wobei einer der zwei Sensoren 40 ein Hall-Sensor ist und der andere der zwei Sensoren 40 ein Lichtsensor ist, und einer der zwei Sender 42 ein Magnet ist, der dem Hall-Sensor zugeordnet ist, und der andere der zwei Sender 42 eine Lichtquelle ist, die dem Lichtsensor zugeordnet ist.

In einem weiteren Ausführungsbeispiel können die zumindest zwei Sensoren 40 mit jeweils voneinander unabhängigen Erfassungsbereichen in einer gemeinsamen Gehäuseanordnung aufgenommen sein. Darüber hinaus können einzeln vorgesehene Sensoren 40 und/oder in einer gemeinsamen Gehäuseanordnung verbaute Sensoren 40 jeweils dampf- und/oder wasserdicht gekapselt, beispielsweise verklebt oder vergossen sein.

Bei der der in Fig. 1 dargestellten Handsteuereinrichtung 10 sind vorzugsweise die zumindest zwei Sensoren 40 ortsfest in dem Griff- oder Handstück aufgenommen und angeordnet, und ist der zumindest eine Sender 42 in dem Hebel 22 beweglich angeordnet und kann durch Betätigung (Niederdrücken bzw. Loslassen) des Hebels 22 auf einer vorbestimmten Bahnkurve auf die zumindest zwei Sensoren 40 zu und von diesen weg bewegt werden. In anderen Worten führt ein Betätigen des Hebels 22 durch Niederdrücken dazu, dass sich der zumindest eine Sender 42 den zumindest zwei Sensoren 40 annähert, so dass diese dessen Ausgangsgröße (in den vorliegenden Ausführungsbeispiel beispielsweise eine magnetische Größe oder einen Lichtstrom) erfassen und beispielsweise bei Überschreiten eines Schwellenwerts schalten oder ihr Ausgangssignal ändern, und führt ein entgegengesetzt wirkendes Betätigen des Hebels 22 durch Loslassen dazu, dass sich der zumindest eine Sender 42 von den zumindest zwei Sensoren 40 entfernt und sich das von diesen erfassbare Signal abschwächt. Die Bewegung des Senders 42 folgt hierbei aufgrund der Hebelgeometrie jeweils auf einer vorbestimmten Bewegungs- oder Bahnkurve.

Bei der der in Fig. 2 dargestellten Handsteuereinrichtung 10 sind vorzugsweise die zumindest zwei Sensoren 40 ortsfest in dem Griff- oder Handstück aufgenommen und angeordnet, und ist der zumindest eine Sender 42 so in dem Verschiebeweg 27 zwischen dem Schieber 23 und der Rückholfeder 25 angeordnet, dass eine Betätigung des Schiebers 23 zu einer Verschiebung des Senders 42 in einer ersten Richtung und ein Loslassen des Schiebers 23 aufgrund der Rückstellkraft der Rückholfeder 25 zu einer Verschiebung des Senders 42 in einer zweiten, der ersten Richtung entgegengesetzten Richtung führt. In anderen Worten wird der zumindest eine Sender 42 bei Betätigung/Loslassen des Schiebers 23 in einer Hin- und Her-Bewegung entlang des Verschiebewegs 27 an die dazu in geeigneter positioneller Beziehung angeordneten, zumindest zwei Sensoren 40 herangeführt bzw. von diesen wegbewegt, so dass diese die Ausgangsgröße des zumindest einen Senders 42 (in den vorliegenden Ausführungsbeispiel beispielsweise eine magnetische Größe oder einen Lichtstrom) erfassen und beispielsweise bei Überschreiten eines Schwellenwerts oder bei Abschwächung der erfassbaren Größe schalten oder ihr Ausgangssignal ändern. Insoweit entspricht auch hier der Verschiebeweg 27 einer vorbestimmten Bahnkurve, entlang welcher der zumindest eine Sender 42 beweglich ist.

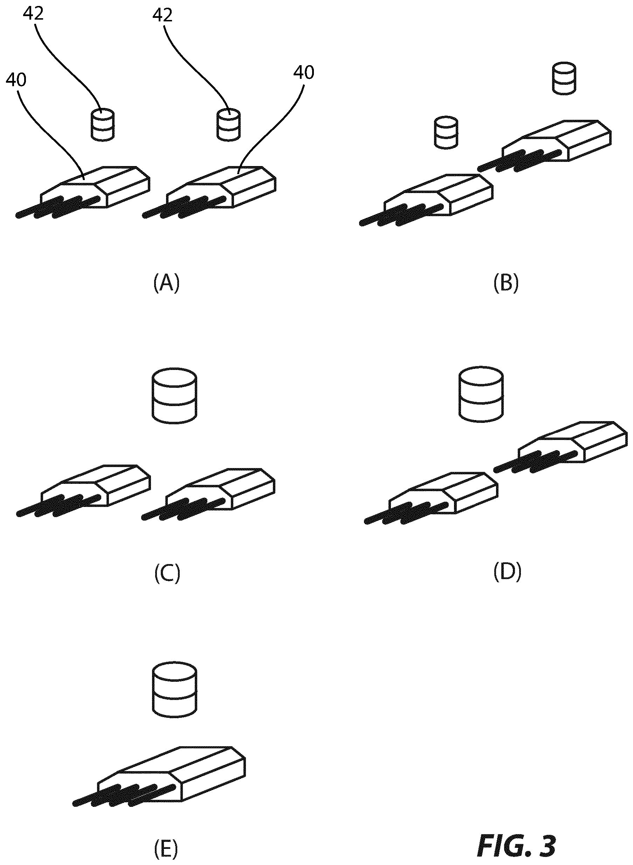

Fig. 3 zeigt eine Prinzipdarstellung eine Anzahl möglicher Sensor-Sender-Anordnungen am Beispiel des auf Magneten und magnetisch beeinflussbaren Sensoren basierenden sensorischen Gebersystems anhand verschiedener Ausführungsbeispiele. Die Anzahl der Anordnungen ist hierbei nicht auf die dargestellte Anzahl beschränkt, so dass sich anwendungsfallbezogen und/oder in einer konkret praktischen Ausführungsform auch andere Ausgestaltungen ergeben können.

Fig. 3(A) zeigt einen Fall, in welchem zwei Sensoren 40 (Hall-Sensoren) und zwei Sender 42 (Magnete) nebeneinander liegend angeordnet sind. Jeder Sender 42 beaufschlagt in diesem Fall einzeln den ihm zugeordneten Sensor 40. Fig. 3(B) zeigt einen Fall, in welchem zwei Sensoren 40 (Hall-Sensoren) und zwei Sender 42 (Magnete) hintereinander liegend angeordnet sind. Auch in diesem Fall beaufschlagt jeder Sender 42 einzeln den ihm zugeordneten Sensor 40. Fig. 3(C) zeigt einen Fall, in welchem zwei Sensoren 40 (Hall-Sensoren) nebeneinander liegend und für beide Sensoren 40 gemeinsam ein Sender 42 (Magnet) angeordnet sind. Der nur eine Sender 42 beaufschlagt in diesem Fall beide ihm zugeordnete Sensoren 40. Fig. 3(D) zeigt einen Fall, in welchem zwei Sensoren 40 (Hall-Sensoren) hintereinander liegend und für beide Sensoren 42 gemeinsam ein Sender 42 (Magnet) angeordnet sind. Auch in diesem Fall beaufschlagt der Sender 42 beide ihm zugeordnete Sensoren 40. Fig. 3(E) schließlich zeigt einen Fall, in dem zwei Sensoren 40 in einem gemeinsamen Sensorgehäuse aufgenommen sind und diesen gemeinsam ein Sender 42 zugeordnet ist.

Es wird angemerkt, dass in Fig. 3(A) bis 3(D), d.h. in den Fällen zweier Sensoren 40 mit zwei Magneten bzw. Sendern 42, als Anschlüsse an jedem Sensor 40 Ground bzw Masse, Supply Voltage bzw. Versorgungsspannung und Output bzw. Ausgang(ssignal) in Form von dazu vorgesehen insgesamt drei Sensoranschlüssen bereitgestellt sein können, und in Fig. 3(E), d.h. einem Doppelsensor mit einem Magneten bzw. Sender 42 als Anschlüsse Ground bzw Masse, Supply Voltage bzw. Versorgungsspannung und Output 1 bzw. Ausgang(ssignal) 1 und Output 2 bzw. Ausgang(ssignal) 2 in Form von dazu vorgesehen insgesamt vier Sensoranschlüssen bereitgestellt sein können.

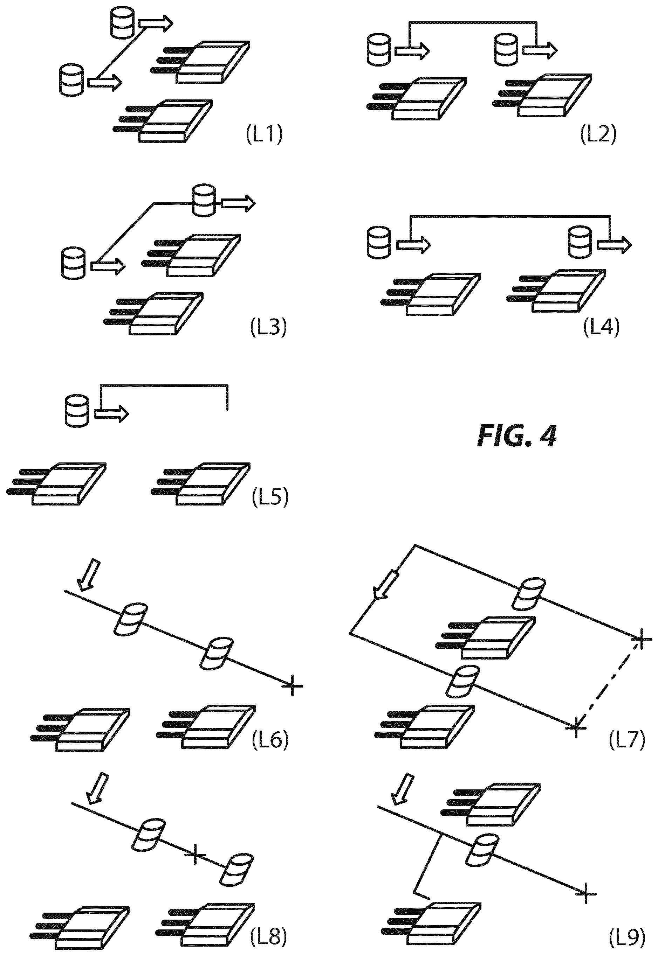

Fig. 4 zeigt eine Prinzipdarstellung von Konzepten für Handsteuereinrichtungen (hier mittels des Hebels 22 oder des Schiebers 23) unter Verwendung der Anordnungen und/oder Ausführungsbeispiele nach Fig. 3.

Im Einzelnen stellen Fig. 4(L1) bis Fig. 4(L5) schieberbasierte (parallel: (L1), (L2); versetzt: (L3), (L4), (L5)) Steuerungen mit jeweils einem oder zwei Sendern 42 dar, bei welchen der zumindest eine beweglich angeordnete Sender 42 vermittels des Schiebers 23 (Fig. 2) der entlang einer gedachten Linie in Richtung eines ebenfalls dargestellten Pfeils (und entgegengesetzt zu diesem), d.h. in Richtung der zumindest zwei ortsfest angeordneten Sensoren 40 bzw. für einen entgegengesetzten Vorgang von diesen weg verschiebbar ist.

Fig. 4(L6) bis (L9) stellen weiter hebelbasierte (parallel: (L6), L(7); versetzt: (L8), L(9)) Steuerungen mit jeweils einem oder zwei Sendern 42 dar, bei welchen der zumindest eine beweglich angeordnete Sender 42 vermittels des Hebels 22 (Fig. 1) in Richtung der zumindest zwei ortsfest angeordneten Sensoren 40 bzw. für einen entgegengesetzten Vorgang von diesen weg verschwenkbar ist. Das in Fig. 4(L6) bis (L9) dargestellte Kreuzsymbol stellt jeweils einen Drehpunkt bzw eine Drehachse des Hebels 22 dar, um welchen der entlang einer gedachten Linie angeordnete zumindest eine Sender 42 in Richtung des ebenfalls dargestellten Pfeils (und entgegengesetzt zu diesem) verschwenkbar ist.

Es wird angemerkt, dass in Fig. 4 jeweils der zumindest eine Sender 42 als beweglich und die zumindest zwei Sensoren 40 als ortsfest angeordnet dargestellt sind. Die Erfindung ist jedoch nicht notwendigerweise darauf beschränkt. Vielmehr ist ebenfalls eine Konfiguration darstellbar, in welcher jeweils der zumindest eine Sender 42 ortsfest und die zumindest zwei Sensoren 40 beweglich angeordnet sind.

Weitere Ausführungsbeispiele beinhalten Kombinationen verschiedener Sensortypen. In anderen Worten können bei den wie vorstehend beschrieben möglichen einzelnen Anordnungssystemen mit zwei Hall-Sensoren als Empfänger bzw. Sensor 40 und zwei Magneten als Sender 42 auch unterschiedliche Sensortypen kombiniert werden. Beispielsweise sind die beiden Hall-Sensoren in den Fällen der Fig. 4 (L1) bis Fig. 4(L9) durch zum Beispiel zwei Lichtsysteme (Lichtsender und Lichtempfänger) ersetzbar, deren Lichtemissionen im sichtbaren Bereich oder unsichtbaren Bereich, beispielsweise dem Infrarotbereich, liegen können. In diesen Fällen kann eine zugeordnete Lichtquelle als der zumindest eine Sender 42 hinter lichtdurchlässigen Werkstoffen, beispielsweise opaken oder klaren Kunststoffen oder Glas, oder auch hinter einem in Bezug auf Transparenz klar bis dunkel ausgebildeten, jedoch für Infrarot durchlässigen Werkstoff verdeckt, abgekapselt und geschützt verbaut sein.

Alternativ kann zumindest in den Fällen der Fig. 4(L1) bis Fig. 4(L4) und Fig. 4(L6) bis Fig. 4(L8), d.h. in den Fällen, in welchen jedem Sensor 40 ein Sender 42 direkt zugeordnet ist und sich daher der Sender 42 nicht von einem zu einem anderen Sensor 40 bewegt, auch nur einer der beiden Hall-Sensoren durch ein solches Lichtsystem ersetzt sein.

Ferner ist es möglich, bei den vorstehend beschriebenen Anordnungen die zumindest zwei Sensoren 40 vollständig in analoger Ausführung, vollständig in digitaler Ausführung oder in einer Kombination aus analoger Ausführung und digitaler Ausführung vorzusehen.

Vorstehend wurde somit ein redundantes Gebersystem bei einer Elektrohandsteuerung beschrieben, die vorzugsweise in Form einer Gehäuseanordnung mit zumindest zwei Sensorelementen und zumindest einem Magneten ausgebildet ist, die damit zumindest zweikanalig ausgeführt sind, um bei mittels zumindest doppelter (redundanter) Abfrage erfassten fehlerhaften Signalen, d.h. einer Fehlfunktion wie beispielsweise einem Start ohne Anforderung, die Funktion der Handsteuereinrichtung anzuhalten und dadurch sicherzustellen, dass eine ungewollte Aktivierung bzw. ein ungewolltes Weiterlaufen von Anwendungsteilen (z.B. im Situs) unterbunden wird. Das Gebersystem kann somit für ein medizinisches und/oder chirurgisches Motorensystem ausgelegt sein und eine mehrkanalig redundant ausgelegte Erfassungseinrichtung beinhalten, die in einer zur Steuerung des medizinischen Motorensystems angepassten Handsteuereinrichtung aufgenommen und dazu angeordnet ist, die Betätigung einer Betätigungseinrichtung der Handsteuereinrichtung zu erfassen, wobei die mehrkanalig redundante Erfassungseinrichtung auf Plausibilität von Ausgangssignalen derselben abfragbar ausgelegt ist.

Es versteht sich, dass den beschriebenen Ausführungsbeispielen und nicht maßstabsgetreuen Zeichnungen lediglich beispielhafter Charakter zukommt und sich insoweit für den Fachmann Modifikationen ohne Weiteres ergeben können, ohne dass dadurch der beschreibungsgemäße Rahmen und der durch die beigefügten Ansprüche definierte Schutzumfang verlassen werden. Ebenso unterliegen äußere Formen, Dimensionen und dergleichen keinen besonderen Beschränkungen, solange durch sie die erfindungsgemäße Wirkung und Funktionalität bereitgestellt und erzielt wird.

## Patentansprüche

1. Handsteuereinrichtung (10) mit einem Gebersystem für ein medizinisches Motorensystem, mit einer mehrkanalig redundant ausgelegten Erfassungseinrichtung (40, 42), die in die zur Steuerung des medizinischen Motorensystems angepassten Handsteuereinrichtung (10) aufgenommen und dazu angeordnet ist, die Betätigung einer Betätigungseinrichtung (22; 23) der Handsteuereinrichtung (10) zu erfassen, wobei
die Betätigungseinrichtung als Hebel oder Schieber konfiguriert ist;
die mehrkanalig redundante Erfassungseinrichtung (40, 42) zumindest zwei Erfassungsempfangseinrichtungen (40) und genau eine Erfassungssendeeinrichtung (42) oder jeweils eine zugeordnete Erfassungssendeeinrichtung (42) beinhaltet;
die genau eine Erfassungssendeeinrichtung allen der zumindest zwei Erfassungsempfangseinrichtungen (40) zugeordnet ist und dazu angeordnet ist, diese mit einer dem Grunde nach gleichen Erfassungssendegröße zu beaufschlagen; oder die für jede der zumindest zwei Erfassungsempfangseinrichtungen (40) zugeordnete Erfassungssendeeinrichtung (42) vorgesehen und dazu angeordnet ist, eine entsprechende der zumindest zwei Erfassungsempfangseinrichtungen (40) mit einer jeweiligen Erfassungssendegröße zu beaufschlagen; und
die mehrkanalig redundante Erfassungseinrichtung (40, 42) auf Plausibilität von Ausgangssignalen derselben abfragbar ausgelegt ist,
**dadurch gekennzeichnet, dass**
bei dem Gebersystem die zumindest zwei Erfassungsempfangseinrichtungen (40) sowie die genau eine Erfassungssendeeinrichtung (42) oder die jeweils eine zugeordnete Erfassungssendeeinrichtung (42) dampf- und/oder wasserdicht vergossen sind.

2. Handsteuereinrichtung (10) nach Anspruch 1, bei dem die zumindest zwei Erfassungsempfangseinrichtungen (40) in Richtung eines Hebelwegs oder Schieberwegs der genau einen Erfassungssendeeinrichtung (42) versetzt angeordnet sind.

3. Handsteuereinrichtung (10) nach Anspruch 1 oder 2, bei dem die zumindest zwei Erfassungsempfangseinrichtungen (40) Hall-Sensoren oder Lichtsensoren und die genau eine Erfassungssendeeinrichtung (42) oder die jeweils eine zugeordnete Erfassungssendeeinrichtung (42) jeweils einen Magneten oder eine Lichtquelle, beinhalten.

4. Handsteuereinrichtung (10) nach einem der vorangehenden Ansprüche, bei dem die zumindest zwei Erfassungsempfangseinrichtungen (40) gleichartige Erfassungsempfangseinrichtungen sind.

5. Handsteuereinrichtung (10) nach einem der Ansprüche 1 bis 3, bei dem die zumindest zwei Erfassungsempfangseinrichtungen (40) unterschiedliche Erfassungsempfangseinrichtungen sind.

6. Handsteuereinrichtung (10) nach einem der vorangehenden Ansprüche, bei dem die zumindest zwei Erfassungsempfangseinrichtungen (40) mit jeweils voneinander unabhängigen Erfassungsbereichen in einer gemeinsamen Gehäuseanordnung aufgenommen sind.

7. Handsteuereinrichtung (10) nach einem der vorangehenden Ansprüche, bei dem die zumindest zwei Erfassungsempfangseinrichtungen (40) ortsfest angeordnet sind, und die zumindest eine Erfassungssendeeinrichtung (42) beweglich angeordnet und durch Betätigung der Betätigungseinrichtung (22; 23) auf einer vorbestimmten Bahnkurve auf die zumindest zwei Erfassungsempfangseinrichtungen (40) zu bewegbar ist.

8. Handsteuereinrichtung (10) nach einem der vorangehenden Ansprüche, bei dem die zumindest zwei Erfassungsempfangseinrichtungen (40) dazu programmiert sind, dasselbe Ausgangssignal auszugeben, oder dazu programmiert sind, unterschiedliche Ausgangssignale auszugeben.

## Claims

1. A hand-operated control device (10) with a detector system for a medical motor system, comprising a multi-channel redundantly-configured detection device (40, 42) which is accommodated in the hand-operated control device (10) that is adapted to control the medical motor system and which is arranged to detect the actuation of an actuating device (22; 23) of the hand-operated control device (10), wherein
the actuation device is configured as a lever or slider;
the multi-channel redundant detection device (40, 42) comprises at least two detection receiving devices (40) and exactly one detection transmitting device (42) or respectively one associated detection transmitting device (42);
the exactly one detection transmitting device is associated with all of said at least two detection receiving devices (40) and arranged to charge said at least two detection receiving devices with a detection transmitting quantity which is basically the same; or the detection transmitting device (42) associated with each of the at least two detection receiving devices (40) is provided and arranged to charge a corresponding one of the at least two detection receiving devices (40) with a respective detection transmitting quantity; and
the multi-channel redundant detection device (40, 42) is designed to allow the plausibility of its output signals to be checked,
**characterized in that**
for the detector system, the at least two detection receiving devices (40) as well as the exactly one detection transmitting device (40) or the respectively one associated detection transmitting device (42) are molded together in a vapor-proof and/or watertight manner.

2. The hand-operated control device (10) according to claim 1, in which the at least two detection receiving devices (40) are positioned so as to be offset in the direction of a lever path or slider path of the exactly one detection transmitting device (42).

3. The hand-operated control device (10) according to claim 1 or 2, in which the at least two detection receiving devices (40) comprise Hall sensors or light sensors and the exactly one detection transmitting device (42) or the respective one associated detection transmitting device (42) respectively comprises a magnet or a light source.

4. The hand-operated control device (10) according to any of the preceding claims, in which the at least two detection receiving devices (40) are identical detection receiving devices.

5. The hand-operated control device (10) according to any of the claims 1 to 3, in which the at least two detection receiving devices (40) are different detection receiving devices.

6. The hand-operated control device (10) according to any of the preceding claims, in which the at least two detection receiving devices (40), each with mutually independent detection ranges, are accommodated in a common housing assembly.

7. The hand-operated control device (10) according to any of the preceding claims, in which the at least two detection receiving devices (40) are stationarily positioned and the at least one detection transmitting device (42) is movably positioned and can be moved toward the at least two detection receiving devices (40) by actuating the actuating device (22; 23) on a predetermined trajectory.

8. The hand-operated control device (10) according to any of the preceding claims, in which the at least two detection receiving devices (40) are programmed to output the same output signal or are programmed to output different output signals.

## Revendications

1. Dispositif de commande manuelle (10) comprenant un système à capteurs pour un système motorisé médical, avec un dispositif de détection (40, 42) à plusieurs canaux conçu de manière redondante, qui est intégré dans le dispositif de commande manuelle (10) adapté pour la commande du système motorisé médical et est disposé pour détecter l'activation d'un dispositif d'actionnement (22 ; 23) du dispositif de commande manuelle (10), dans lequel
le dispositif d'actionnement est configuré comme levier ou coulisseau ;
le dispositif de détection (40, 42) redondant à plusieurs canaux comporte au moins deux dispositifs de réception de détection (40) et exactement un dispositif d'émission de détection (42) ou respectivement un dispositif d'émission de détection (42) associé ;
l'exactement un dispositif d'émission de détection est associé à tous les dispositifs de réception de détection (40) et est disposé pour les solliciter avec une taille d'émission de détection fondamentalement égale ; ou le dispositif d'émission de détection (42) associé à chacun des au moins deux dispositifs de réception de détection (40) est prévu et disposé pour solliciter un dispositif correspondant desdits au moins deux dispositifs de réception de détection (40) avec une taille d'émission de détection respective ; et
le dispositif de détection (40, 42) redondant à plusieurs canaux est conçu de manière à pouvoir être interrogé sur la plausibilité des signaux de sortie de celui-ci,
**caractérisé en ce que**
dans le système à capteurs, lesdits au moins deux dispositifs de réception de détection (40) ainsi que l'exactement un dispositif d'émission de détection (42) ou le dispositif d'émission de détection (42) associé respectif sont scellés de manière étanche à la vapeur et/ou à l'eau.

2. Dispositif de commande manuelle (10) selon la revendication 1, pour lequel lesdits au moins deux dispositifs de réception de détection (40) sont disposés de manière décalée dans la direction d'un trajet de levier ou d'un trajet de coulisseau dudit exactement un dispositif d'émission de détection (42).

3. Dispositif de commande manuelle (10) selon la revendication 1 ou 2, pour lequel lesdits au moins deux dispositifs de réception de détection (40) comportent des capteurs de Hall ou des capteurs de lumière et ledit exactement un dispositif d'émission de détection (42) ou ledit dispositif d'émission de détection (42) associé respectif comportent un aimant ou une source lumineuse.

4. Dispositif de commande manuelle (10) selon l'une quelconque des revendications précédentes, pour lequel lesdits au moins deux dispositifs de réception de détection (40) sont des dispositifs de réception de détection identiques.

5. Dispositif de commande manuelle (10) selon l'une quelconque des revendications 1 à 3, pour lequel lesdits au moins deux dispositifs de réception de détection (40) sont des dispositifs de réception de détection différents.

6. Dispositif de commande manuelle (10) selon l'une quelconque des revendications précédentes, pour lequel lesdits au moins deux dispositifs de réception de détection (40) sont intégrés avec des zones de détection respectivement indépendantes les unes des autres dans un ensemble formant boîtier commun.

7. Dispositif de commande manuelle (10) selon l'une quelconque des revendications précédentes, pour lequel lesdits au moins deux dispositifs de réception de détection (40) sont disposés fixement, et ledit au moins un dispositif d'émission de détection (42) est disposé de manière mobile et par actionnement du dispositif d'actionnement (22 ; 23) peut être déplacé vers une trajectoire prédéterminée sur lesdits au moins deux dispositifs de réception de détection (40).

8. Dispositif de commande manuelle (10) selon l'une quelconque des revendications précédentes, pour lequel lesdits au moins deux dispositifs de réception de détection (40) sont programmés pour émettre le même signal de sortie, ou sont programmés pour émettre des signaux de sortie différents.
